# FASCICULE DE BREVET EUROPEEN

(11) **EP 1 444 973 B1**
(45) Date de publication et mention de la délivrance du brevet: **31.05.2006**
(21) Numéro de dépôt: 04300042.1
(22) Date de dépôt: 26.01.2004
(51) Int. Cl.: A61K 8/81, A61K 8/06, A61Q 1/00

(54) **Poudre homogène consistant en un mélange de latex inverse auto-inversible et d'une poudre à usage cosmétique ou pharmaceutique, et son utilisation comme agent de texture**
Homogenes Pulver aus einem selbstdispergierenden Inverslatex und einem Pulver zur kosmetischen oder pharmakologischen Verwendung, sowie seine Verwendung als Texturmittel
Homogeneous powder consisting in a mixture of an autodispersing reverse latex and a powder for cosmetic or pharmaceutical use, and its use as a texturing agent

(30) Priorité: 06.02.2003 FR 0301395
(43) Date de publication de la demande: 11.08.2004
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Roso, Alicia, 81710 Saix (FR); Bulcourt, Catherine, 92100 Boulogne Billancourt (FR); Michel, Nelly, 94700 Maisons Alfort (FR)
(74) Mandataire: Conan, Philippe Claude

(56) Documents cités:
- EP-A- 1 055 707
- WO-A-01/35922
- US-B1- 6 274 151
- US-B1- 6 296 859

## Description

L'invention a pour objet de nouvelles poudres homogènes ainsi que leur utilisation comme agent de texture dans des applications cosmétiques, dermopharmaceutiques, pharmaceutiques, dans le traitement du papier ou des textiles.

Les agents de texture sont fréquemment utilisés dans la fabrication des formulations destinées au soin ou au maquillage de la peau ou des muqueuses ou bien en application sur des supports tels que le papier ou les textiles. Leur fonction principale est d'améliorer les propriétés sensorielles et rhéologiques des formulations dans lesquelles ils sont incorporés ou des supports sur lesquels ils sont appliqués. Comme exemples d'agents de textures utilisés en cosmétique, il y a les poudres de polyméthacrylate de méthyle (MICROPEARL™), les poudres de polyamide (NYLON™), les poudres de silicone (DC9506™, POLYTRAP™), les amidons modifiés (DRY FLO™). Certaines de ces poudres procurent à l'utilisateur, une sensation de douceur à l'étalement et un toucher poudré persistant ; d'autres inhibent la sensation grasse ressentie à l'étalement et engendrent un effet matifiant pendant une longue durée.

C'est ainsi que la demande de brevet européen EP 1 055 707 A1 en son exemple 25, la demande internationale WO 01/35922 et le brevet américain US 6,296,859 B1 , en leurs exemples 22, divulguent un fond de teint fluide contenant 10 % en poids de pigments et de charges minérales, et 1,2% en poids d'un latex inverse auto-inversible

Certains dérivés d'aminoacides, comme la N-lauroyl lysine, sont parfois ajoutés dans les formules de maquillage, pour combiner les effets de douceur à l'application et de bonne tenue sur la peau. Le brevet américain US 6,274,151 B1 divulgue en son exemple 34 une poudre pressée comprenant 57 % de talc et 3% d'une composition comprenant environ 45% de palmitoyl proline, de 10% à 15% d'acide N-palmitoyl glutamique, de 10% à 15% de N-palmitoyl sarcosine et environ 30% d'acide palmitique

Cet effet peut également être obtenu grâce à un traitement de surface des poudres par divers composés dont les aminoacides.

Dans certains cas, on ajoute aussi dans les formulations, des fibres d'origine naturelle, comme les fibres de cellulose ou de coton ou des fibres synthétiques comme des fibres de polyéthylène, de téflon ou de polyester afin d'en modifier les caractéristiques rhéologiques, d'améliorer l'homogénéité de leur répartition sur la surface à enduire ainsi que leur tenue sur cette même surface.

Certaines charges comme le talc, le mica, la séricite ou encore des charges composites sont également utilisées pour moduler les propriétés lubrifiantes de la formulation et faciliter l'écoulement ou l'étalement sur le support.

D'autres types de charges pigmentaires telles que l'oxyde de titane, l'oxyde de zinc ou les oxydes de fer peuvent aussi être incorporés dans ces formulations pour en moduler la transparence ou la couleur à l'application, tout en influant sur sa texture finale.

Ces poudres sont, en général, bien adaptées à la fabrication de formulations de type poudre libre ou poudre compacte ou de formulations à phase grasse continue, comme les émulsions eau - dans - huile, les émulsions eau dans huile de silicone, les sticks et autres formules compactes.

Par contre, elles sont souvent difficiles à mettre en oeuvre dans les milieux à phase aqueuse continue, comme les lotions, les gels, les gels-crème ou les émulsions huile - dans - eau. Il faut alors réaliser des études préalables spécifiques et coûteuses, pour chaque poudre et chaque type de formulations pour obtenir à la fois une bonne dispersion de la poudre et une bonne stabilité de la formulation.

Le formulateur est alors souvent obligé d'utiliser, soit des microsphères microporeuses hydrophiles de type MICROPEARL™ associées à des agents stabilisants, soit des poudres traitées en surface pour améliorer la compatibilité avec les autres ingrédients de la formulation. Cependant, dans ce dernier cas, le traitement adapté est spécifique à la formulation choisie qui, de plus, ne dispense pas le formulateur d'une étude de stabilité de la poudre traitée choisie au sein de la formulation. Enfin, cette dernière solution n'est généralement pas adaptée aux formulations de type phase continue aqueuse que ce soit en l'absence ou en présence d'un faible taux de phase grasse.

C'est pourquoi dans le cadre de ses recherches sur l'amélioration de la texture des formulations, la demanderesse a cherché à mettre au point de nouveaux agents de texture sous forme de poudres, polyvalents, faciles à mettre en oeuvre, aussi bien dans des formulations solides de type poudres libres ou poudres compactées, que dans des formulations à phase grasse continue ou que dans des formulations à phase aqueuse continue contenant ou non un faible taux de phase grasse.

L'invention a pour objet une poudre homogène consistant essentiellement en un mélange contenant :
- de 5% à 80 % en poids d'au moins une composition comprenant une phase huile, une phase aqueuse, au moins un agent émulsionnant de type eau dans huile (E/H), au moins un agent émulsionnant de type huile dans eau (H/E), comprenant de 20% à 70% en poids, et de préférence de 25% à 50% en poids, d'un polyélectrolyte branché ou réticulé, caractérisé en ce que ledit polyélectrolyte est, soit un homopolymère à base d'un monomère possédant ou bien une fonction acide fort partiellement ou totalement salifiée, ou bien une fonction acide faible partiellement ou totalement salifiée, soit un copolymère à base d'au moins un monomère possédant une fonction acide fort, copolymérisé ou bien avec au moins un monomère possédant une fonction acide faible ou bien avec au moins un monomère neutre, soit un copolymère à base d'au moins un monomère possédant une fonction acide faible, copolymérisé avec au moins soit un monomère neutre, soit un monomère possédant une fonction acide faible. ;
- de 95 % à 20% en poids du mélange d'au moins une poudre cosmétiquement ou pharmaceutiquement acceptable.

Par "agent émulsionnant du type eau-dans-huile", on désigne des agents tensioactifs possédant une valeur de HLB suffisamment faible pour fournir des émulsions eau dans huile tels que les polymères tensioactifs du type copolymères séquencés polyéthylèneglycol poly(acide hydroxy stéarique), commercialisés sous le nom HYPERMER™, tels que les esters de sorbitan, comme le mono-oléate de sorbitan commercialisé par la demanderesse sous le nom MONTANE™ 80, l'isostéarate de sorbitan commercialisé par la demanderesse sous le nom MONTANE™ 70, l'oléate de sorbitan éthoxylé avec 5 moles d'oxyde d'éthylène (5 OE) commercialisé par la demanderesse sous le nom MONTANE™ 81, l'alcool oléocétylique diéthoxylé (2 OE), commercialisé par la demanderesse sous le nom SIMULSOL™ OC 72 ou le sesquioléate de sorbitan commercialisé par la demanderesse sous le nom MONTANE™ 83.

Par "agent émulsionnant du type huile-dans-eau", on désigne des agents tensioactifs possédant une valeur de HLB suffisamment élevée pour fournir des émulsions huile dans l'eau tels que les esters de sorbitan éthoxylés comme l'oléate de sorbitan éthoxylé avec 20 moles d'oxyde d'éthylène (20 OE), commercialisé par la demanderesse sous le nom MONTANOX™ 80, l'huile de ricin éthoxylée à 40 moles d'oxyde d'éthylène (40 OE), commercialisée par la demanderesse sous le nom SIMULSOL™ OL 50, le laurate de sorbitan éthoxylé à 20 moles d'oxyde d'éthylène (20 OE), commercialisé par la demanderesse sous le nom MONTANOX™ 20, le trioléate de sorbitan éthoxylé à 25 moles commercialisé par la demanderesse sous le nom MONTANOX™ 85, l'alcool laurique éthoxylé à 7 moles d'oxyde d'éthylène (7 OE), commercialisé par la demanderesse sous le nom SIMULSOL™ P 7, l'alcool oléocétylique décaéthoxylé (10 OE), commercialisé par la demanderesse sous le nom SIMULSOL™ OC 710 ou les hexaoléoates de sorbitan polyéthoxylés commercialisé sous les noms G-1086™ et G- 1096™.

Par polymère branché, on désigne un polymère non linéaire qui possède des chaînes pendantes de manière à obtenir, lorsque ce polymère est mis en solution dans l'eau, un fort état d'enchevêtrement conduisant à des viscosités à bas gradient de vitesse très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique.

La fonction acide fort du monomère en comportant est notamment la fonction acide sulfonique ou la fonction acide phosphonique, partiellement ou totalement salifiée. Ledit monomère peut être par exemple l'acide styrènesulfonique partiellement ou totalement salifié ou de préférence l'acide 2-méthyl 2-[(1-oxo 2-propènyl) amino] 1-propanesulfonique partiellement ou totalement salifié, notamment sous forme, soit d'un sel de métal alcalin tel que par exemple le sel de sodium ou le sel de potassium, soit d'un sel d'ammonium, soit le sel d'un amino alcool tel que par exemple le sel de monoéthanolamine ou soit le sel d'un aminoacide tel que par exemple le sel de lysine.

La fonction acide faible du monomère en comportant est notamment la fonction acide carboxylique, et de préférence, ledit monomère est choisi parmi l'acide acrylique, l'acide méthacrylique, l'acide itaconique, l'acide maléique ou l'acide 3-méthyl 3-[(1-oxo 2-propènyl) amino] butanoïque, lesdits acides étant partiellement ou totalement salifiés notamment sous forme, soit d'un sel de métal alcalin tel que par exemple le sel de sodium ou le sel de potassium, soit d'un sel d'ammonium, soit le sel d'un amino alcool tel que par exemple le sel de monoéthanolamine ou soit le sel d'un aminoacide tel que par exemple le sel de lysine.

Le monomère neutre est notamment choisi parmi l'acrylamide, le méthacrylamide, le diméthyl acrylamide, l'acrylate de (2-hydroxy éthyle), l'acrylate de (2,3-dihydroxy propyle), le méthacrylate de (2-hydroxy éthyle), le méthacrylate de (2,3-dihydroxy propyle), le diacétone acrylamide ou un dérivé éthoxylé de poids moléculaire compris entre 400 et 1000, de chacun de ces esters.

L'invention a plus particulièrement pour objet une poudre homogène telle que définie précédemment, caractérisée en ce que le polyélectrolyte anionique est réticulé et/ou branché avec un composé diéthylènique ou polyéthylènique dans la proportion molaire exprimée par rapport aux monomères mis en oeuvre, de 0,005% à 1 %, et de préférence de 0,01 % à 0,5 % et, plus particulièrement de 0,01 % à 0,25 %, de préférence celle pour laquelle l'agent de réticulation et/ou l'agent de ramification est choisi parmi le diméthacrylate d'éthylèneglycol, l'acide diallyloxacétique ou un de ses sels comme le le diallyloxyacétate de sodium, le diacrylate d'éthylèneglycol, le diallyl urée, le triallyl amine, le triméthylol propanetriacrylate ou le méthylène-bis-(acrylamide) ou un mélange de ses composés.

La composition mise en oeuvre dans la présente invention contient généralement de 2,5% à 15% en poids, et de préférence de 4% à 9% en poids, d'agents émulsionnants, parmi lesquels de 20% à 50%, notamment de 25% à 40% du poids total des agents émulsionnants présents, sont du type eau dans huile (E/H) et dans laquelle de 80% à 50%, notamment de 75% à 60%, du poids total des agents émulsionnants, sont du type huile dans eau (H/E).

Dans la composition mise en oeuvre dans la présente invention contient généralement, la phase huile représente de 15% à 40% et de préférence de 20% à 25%, de son poids total.

Cette phase huile est généralement constituée soit par une huile minérale commerciale contenant des hydrocarbures saturés comme les paraffines, les isoparaffines, les cycloparaffines, présentant à température ambiante, une densité entre 0,7 et 0,9 et un point d'ébullition supérieur à 180°C, telle que par exemple, l'ISOPAR™ L, l'ISOPAR™M, l'EXXSOL™ D 100 S, ou le MARCOL™52 commercialisés par EXXON CHEMICAL, l'isohexadécane ou l'isododécane, soit par une huile végétale, soit par des esters de glycérol, comme le SOFTENOL™3108, SOFTENOL™3178, SOFTENOL™3100, SOFTENOL™3107, SOFTENOL™3118, soit par des esters d'acides gras, soit une huile de synthèse, soit par un mélange de plusieurs de ces huiles.

Selon un aspect préféré de la présente invention, la phase huile est constituée de MARCOL™ 52, de squalane, de polyisobutène hydrogéné, de palmitate d'octyle, d'isostéarate d'isostéaryle, d'isododécane ou d'isohexadécane ; l'isohexadécane, qui est identifié dans Chemical Abstracts par le numéro RN = 93685-80-4, est un mélange d'isoparaffines en C₁₂, C₁₆ et C₂₀ contenant au moins 97 % d'isoparaffines en C₁₆, parmi lesquelles le constituant principal est le 2,2,4,4,6,8,8-heptaméthyl nonane (RN = 4390-04-9). Il est commercialisé en France par la société BAYER. Le MARCOL™ 52 est une huile commerciale répondant à la définition des huiles de vaseline du Codex français. C'est une huile blanche minérale conforme aux réglementations FDA 21 CFR 172.878 et CFR 178.3620 (a) et elle est inscrite à la Pharmacopée des USA, US XXIII (1995) et à la Pharmacopée européenne (1993). Le SOFTENOL™ 3819, est un mélange de triglycérides d'acides gras comportant de 6 à 10 atomes de carbone. Le SOFTENOL™ 3108 est un mélange de triglycérides d'acides gras comportant de 8 à 10 atomes de carbone. Le SOFTENOL™ 3178 est un mélange de triglycérides d'acides gras comportant de 8 à 18 atomes de carbone. Le SOFTENOL™ 3100 est un mélange de triglycérides d'acides gras comportant de 12 à 18 atomes de carbone. Le SOFTENOL™ 3107 est un mélange de triglycérides d'acides gras comportant 7 atomes de carbone commercialisé sous le nom. Le SOFTENOL™ 3114 est un mélange de triglycérides d'acides gras comportant 14 atomes de carbone. Le SOFTENOL™ 3118 est un mélange de triglycérides d'acides gras comportant 18 atomes de carbone.

Les compositions mises en oeuvre dans la présente invention contiennent généralement entre 20 % et 50 % d'eau. Ils peuvent également contenir divers additifs tels que des agents complexants, des agents de transfert ou des agents limiteurs de chaîne.

De telles compositions sont décrites dans les demandes de brevet français et brevets français publiés sous les numéros 2721511, 2773805, 2774688, 2774996, 2782086, 2785801, 2786493, 2787457, 2789395, 2794034, 2794124, 2808446, 2808447 et 2810883.

Par poudre cosmétiquement ou pharmaceutiquement acceptable, on désigne notamment des poudres d'origine synthétique ou naturelle, minérales ou organiques, hydrophiles ou hydrophobes, de diamètre moyen compris entre environ 0,01 µm et environ 250µm et de préférence entre 1 et 50µm, micronisées ou non, et de toutes formes notamment sous formes de fibre, sous forme lamellaire ou sous forme sphériques, ayant subies éventuellement un traitement de surface.

Il y a par exemple les copolymères d'acide acrylique et méthacrylique ou de leurs esters, les amidons, les silices, les silicates de calcium de magnésium, de baryum, le phosphate de calcium, le nitrure de bore, la lauroyl lysine, les poudres de résine silicone, les carbonates de calcium ou de magnésium, les oxydes de titane ou de zinc ou de Cérium, les oxydes de fer et autres pigments minéraux ou organiques ou les mélanges de ces poudres.

Comme fibres il y a par exemple les fibres naturelles telles que les fibres de coton, de cellulose, de chitosan, les fibres synthétiques telles que les fibres de polyamide comme les Nylon™, les fibres de rayonne™, les fibres de viscose™, d'acétate de cellulose, de poly-p-phénylène téréphtamide comme les Kevlar™, les fibres de polyéthylène ou polypropylène, les fibres de verre, les fibres de carbone, les fibres de téflon™, les fibres de polyester, de polychlorure de vinyle, d'alcool polyvinylique, de polyacrylonitrile, de polyuréthane ou de polyéthylène phtalate.

Comme poudres sous forme lamellaire, il y a par exemple, les talcs, les micas, les mica-titane ou la séricite.

Comme poudres sous forme sphérique, il y a par exemple les polyméthylméthacrylates, souvent désignés dans la littérature par PMMA et qui sont formés de microsphères microporeuses de surface spécifique supérieure ou égale à 0,5 m² par gramme, comme celles commercialisées sous les noms MICROPEARL™ M305, MICROPEARL™ M100, MICROPEARL™ M201 MICROPEARL™ M310; les copolymères ou les terpolymères du méthacrylate de méthyle avec un ou plusieurs monomères choisis parmi l'acrylate de butyle, l'acrylate de (1-méthyl propyle), l'acrylate de (2-méthyl propyle), l'acrylate de (1,1-diméthyl éthyle), le méthacrylate de butyle, le méthacrylate de (1-méthyl propyle), le méthacrylate de (2-méthyl propyle) ou le méthacrylate de (1,1-diméthyl éthyle), tels que ceux commercialisés sous le nom MICROSPHERE™ ;
les microsphères de silice comme celles commercialisées sous les noms Silica beads™ ou Polytrap™ ;
les microsphères creuses en matériau thermoplastique comme les polyéthylènes, les polystyrènes, les polyacrylonitriles ou les polyamides, comme celles commercialisées sous les noms Orgasol™ ou encore en polyesters comme celles commercialisées sous le nom Expancel™ ;
les microcapsules en matériau organique ou inorganique comme celles commercialisées sous le nom Macrolite™.

Selon un premier aspect préféré de la présente invention, le polyélectrolyte présent dans la composition telle que définie précédemment, est choisi parmi les polyélectrolytes suivants :
copolymère d'acide acrylique partiellement salifié sous forme de sel de sodium et d'acrylamide, réticulé au méthylène bis(acrylamide) ;
copolymère d'acide 2-méthyl 2-[(1-oxo 2-propènyl) amino] 1 -propanesulfonique partiellement salifié sous forme de sel de sodium et d'acrylamide, réticulé au méthylène bis(acrylamide) ;
copolymère d'acide 2-méthyl 2-[(1-oxo 2-propènyl) amino] 1-propanesulfonique partiellement salifié sous forme de sel de sodium et d'acide acrylique partiellement salifié sous forme de sel de sodium, réticulé au méthylène bis(acrylamide) ;
copolymère d'acide 2-méthyl 2-[(1-oxo 2-propènyl) amino] 1-propanesulfonique partiellement salifié sous forme de sel de sodium et d'acrylate de 2-hydroxy éthyle, réticulé au méthylène bis(acrylamide) ;
homopolymère d'acide 2-méthyl 2-[(1-oxo 2-propènyl) amino] 1-propanesulfonique partiellement salifié sous forme de sel de sodium réticulé au méthylène bis(acrylamide) ;
homopolymère d'acide acrylique partiellement salifié sous forme de sel d'ammonium ou de sel de monoéthanol amine, réticulé au diallyloxyacétate de sodium ou
homopolymère d'acide acrylique partiellement salifié sous forme de sel d'ammonium ou de sel de monoéthanol amine, réticulé au triallyl amine.

Parmi les latex inverses auto-inversibles définis ci-dessus on peut citer les compositions commercialisées sous les marques SEPIGEL™ 305 (nom INCI : Polyacrylamide and C13-C14 Isoparaffin and Laureth-7), SEPIGEL™ 501 (nom INCI : Acrylamides Copolymer and Mineral oil and Paraffin liquidum and C13-C14 Isoparaffin and Polysorbate 85), SEPIGEL™ 502 (nom INCI : C13-C14 Isoparaffin and Isostearyl isostearate and Sodium polyacrylate and Polyacrylamide and Polysorbate 60), SIMULGEL™ EG (nom INCI : Sodium acrylate and Acryloyldimethyl Taurate Copolymer and Isohexadecane and Polysorbate 80), SIMULGEL™ NS (nom INCI : Hydroxyethyl Acrylate and Sosium Acryloyldimethyl Taurate Copolymer and Squalane and Polysorbate 60), SIMULGEL™ A (nom INCI : Ammonium Polyacrylate and Isohexadecane and Polysorbate 89), SIMULGEL™ 600 (nom INCI : Acrylamide and Sodium Acryloyldimethyl Taurate Copolymer and Polysorbate 80), SIMULGEL™ 800 (nom INCI : Sodium Polyacryloyldimethyl Taurate and Isohexadecane and Sorbitan oleate), SIMULGEL™ HT (Polyacrylamide and paraffin oil and Polysorbate 80), SIMULGEL EPG™ (nom INCI : Sodium Acrylate and Acryloyldimethyl Taurate copolymer and Polyisobutene and Caprylyl capryl Glucoside).

Selon un deuxième aspect particulier de la présente invention, celle-ci a pour une composition telle que définie précédemment dans laquelle la poudre cosmétiquement ou pharmaceutiquement acceptable est choisie parmi les poudres sous forme sphérique, comme par exemple les polyméthylméthacrylates, souvent désignés dans la littérature par PMMA et qui sont formés de microsphères microporeuses de surface spécifique supérieure ou égale à 0,5 m² par gramme, comme celles commercialisées sous les noms MICROPEARL™ M305 , MICROPEARL™ M100, MICROPEARL M310™, MICROPEARL MHB™, MICROPEARL M201™ ; les copolymères ou les terpolymères du méthacrylate de méthyle avec un ou plusieurs monomères choisis parmi l'acrylate de butyle, l'acrylate de (1-méthyl propyle), l'acrylate de (2-méthyl propyle), l'acrylate de (1,1-diméthyl éthyle), le méthacrylate de butyle, le méthacrylate de (1-méthyl propyle), le méthacrylate de (2-méthyl propyle) ou le méthacrylate de (1,1-diméthyl éthyle), tels que ceux commercialisés sous le nom MICROSPHERE™ ou SEPIPRESS™ M.

Selon un troisième aspect particulier de la présente invention la poudre homogène contient au moins 50 % en poids de poudre cosmétiquement ou pharmaceutiquement acceptable telle que définie précédemment.

L'invention a aussi pour objet un procédé de préparation de la poudre homogène telle que définie précédemment, par simple mélange du latex inverse auto-inversible avec la poudre cosmétiquement ou pharmaceutiquement acceptable.

Les poudres homogènes objet de la présente invention sont utilisées comme agents de texture des formulations, cosmétiques ou pharmaceutiques qu'elles soient liquides ou solides. Leurs propriétés physiques et sensorielles, qu'il s'agisse de leur toucher très doux, amélioré par rapport à la poudre utilisée seule, ou de leur excellente adhérence sur la peau, supérieure à celle de la poudre utilisée seule, ainsi que leur aptitude à se mettre en suspension de façon homogène dans les formulations finales, les rendent particulièrement appropriées à une mises en oeuvre dans les formulations solides tels que les fonds de teint, les poudres de maquillages les mascaras ou les rouges à lèvres. Dans le cas de leur mise en oeuvre dans des formulations liquides, il peut s'agir notamment d'émulsions, de lotions ou de gels et plus particulièrement de formulations vaporisables ("sprayables" en langue anglaise) ou encore de solutions imprégnées sur tissus ou papier et plus particulièrement sur des lingettes ou sur des papiers correcteurs de teint.

Les exemples suivants illustrent l'invention sans toutefois la limiter.

### A) Mise en évidence des différences de comportement entre les poudres homogènes selon l'invention et les agents de texture classiques sous forme poudre:

### (1) - Propriétés de mise en suspension - Stabilité de la dispersion aqueuse

On a préparé une composition (1) selon l'invention en mélangeant par simple agitation du MICROPEARL™ M310 et du SIMULGEL™ EG dans un rapport pondéral 60 / 40, puis comparé ses propriétés avec le MICROPEARL™ M310 seul (poudre témoin (t₁)) et avec une formulation équivalente préparée par incorporation successive du MICROPEARL™ M310 et du SIMULGEL™ EG (témoin (t2) = état de la technique).

Pour ce faire, on a préparé des dispersions aqueuses de la poudre homogène selon l'invention et de la poudre témoin (t₁) à 2 % en poids dans l'eau par agitation mécanique avec une turbine défloculeuse.

La préparation témoin (t₂) contenant la même proportion pondérale en MICROPEARL™ M310 et en SIMULGEL™ EG que la composition (1) ces con stituants ayant été ajoutés successivement, est également préparée avec la même agitation mécanique au moyen d'une turbine défloculeuse.

Les observations et analyses des dispersions sont consignées dans le tableau suivant :

| | témoin (t₁) | témoin (t₂) | composition (1) |
|---|---|---|---|
| Aspect visuel | blanc et hétérogène | blanc opaque et homogène | blanc opaque et homogène |
| Aspect microscopique (x 400) | Agglomération de particules (Voir Figure la) | Agglomération de particules (Voir Figure 1b) | Bonne dispersion des particules (Voir Figure 1c) |
| Viscosité Brookfield LVT (Mobile :2 ; Vitesse : 6 | < 50 mPa.s | | 450 mPa.s |
| Stabilité à 25°C | précipite après 24 h | | stable 1 an |
| Stabilité à 40°C | précipite après 24 h | | stable plus de 6 mois |
| Stabilité à 50°C | précipite après 24 h | | stable plus d'un mois |
| Toucher | impossible d'évaluer le toucher -produit déphasé | Etalement facile ; | Etalement facile ; |
| | | Toucher frais et très doux ; | Toucher frais et très doux; |
| | | Effet poudré résiduel | Effet poudré résiduel |

**(2) -** On a préparé une poudre homogène selon l'invention en effectuant un simple mélange de MICROPEARL™ M310 et de SIMULGEL™ EG dans un rapport pondéral 80 / 20 puis comparé ses propriétés avec une dispersion de MICROPEARL™ M310 seul (témoin t₁) et avec la préparation équivalente en % poids consistant en l'ajout successif du MICROPEARL™ M310 et du SIMULGEL™ EG (préparation témoin t₃ = état de la technique). Pour ce faire, on a préparé des dispersions aqueuses de la poudre homogène selon l'invention (composition 2) et de la poudre témoin (poudre t₁) à différentes concentrations dans l'eau par agitation mécanique avec une turbine défloculeuse ainsi que les préparations équivalentes en % poids contenant le MICROPEARL™ M310 et le SIMULGEL™ EG ajoutés successivement (Selon un procédé d'agitation identique).

Les résultats de l'observation et de l'analyse des dispersions sont consignés dans le tableau suivant :

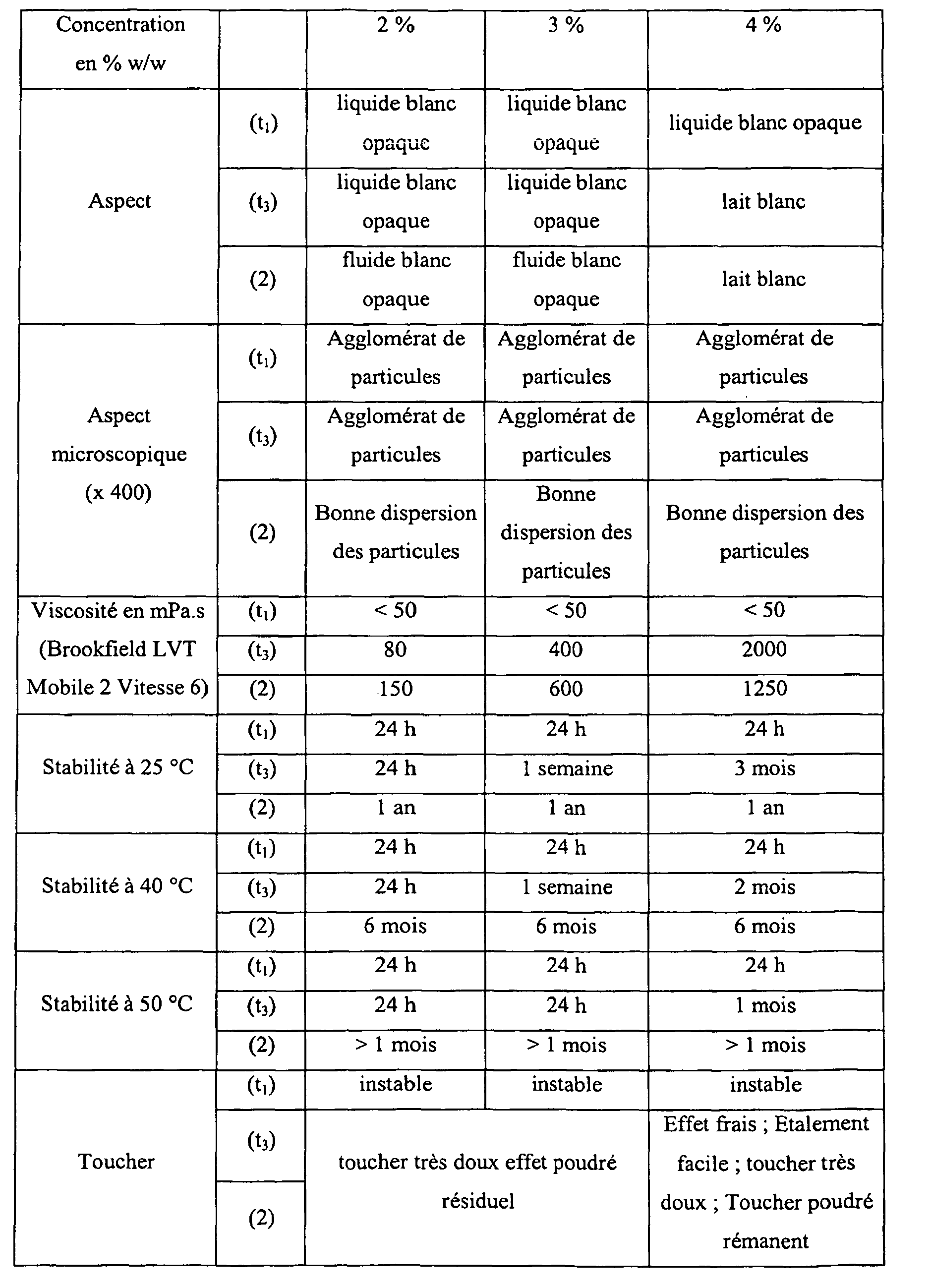

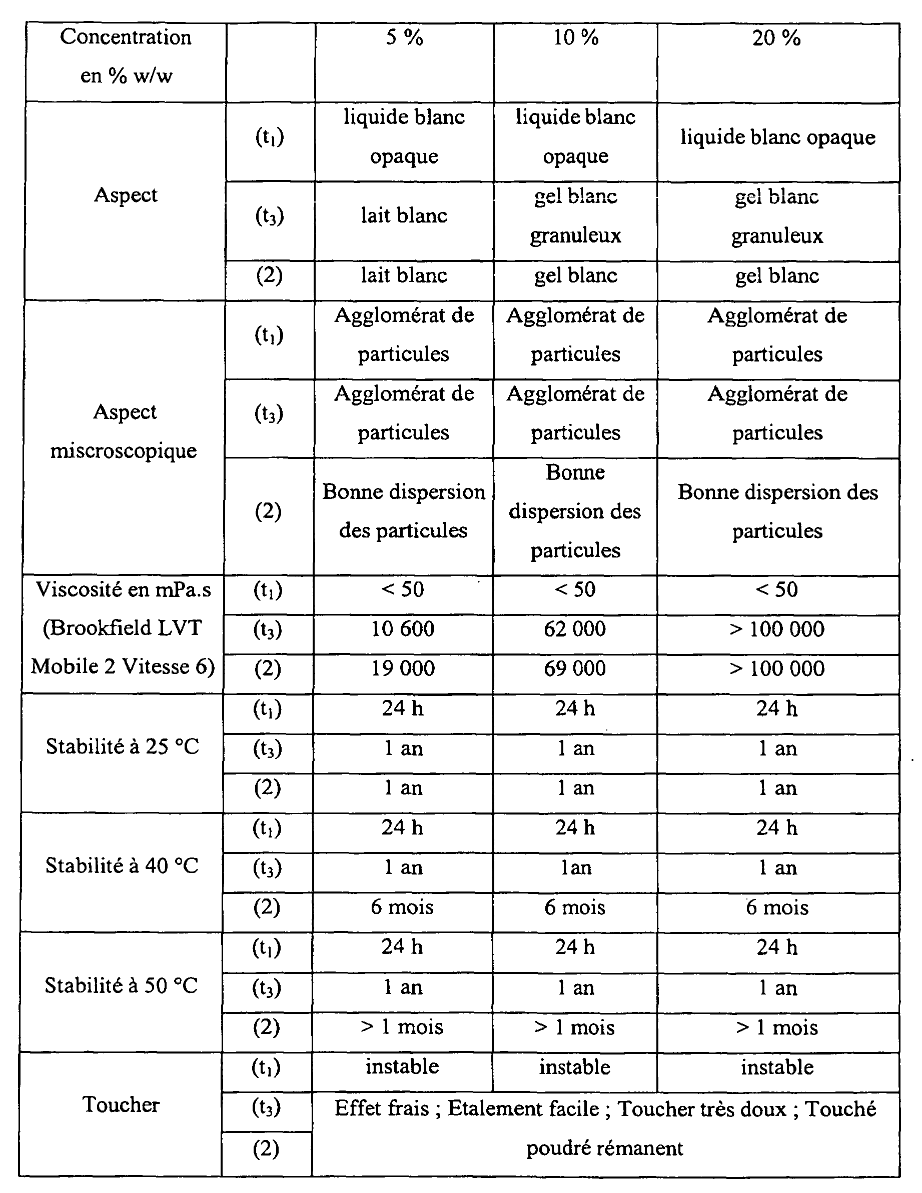

Les poudres homogènes selon l'invention permettent de formuler très simplement, avec un seul ingrédient, des formules avec un toucher remarquable, une excellente stabilité au stockage et une viscosité parfaitement modulable. Les compositions selon l'invention présentent l'avantage d'offrir une parfaite dispersion de la poudre homogène contrairement aux formulations résultant de l'introduction successive du MICROPEARL™ et du SIMULGEL™, même pour des doses de poudre importantes. La stabilité des formulations est également significativement améliorée pour les doses de poudre les plus faibles.

Ces dispersions peuvent être avantageusement utilisées pour tous types de formules de soin ou de maquillage en phase continue aqueuse seules, et cela que les poudres initiales soient hydrophiles, comme le MICROPEARL™ M305 ou hydrophobes, comme le MICROPEARL™ M310.

### (3) - Effet stabilisant au sein d'une émulsion eau dans silicone

On a préparé une série d'émulsions dans des huiles de silicone sur la base de la formule suivante :

| Phase A : | |
|---|---|
| DC5225C™ | 20 % en poids |
| DC345™ | 10 % en poids |
| Sepicide™ HB | 0,3 % en poids |
| Poudre (3) (MICROPEARL™ M310 + SIMULGEL™ EG rapport pondéral 8/2)) | x % en poids ou |
| Poudre témoin (t) (MICROPEARL™ M310) | x % en poids |

| Phase B | |
|---|---|
| Eau | qsp 100% |
| Sepicide™ CI | 0,2 % en poids |
| Glycérine | 5 % en poids |
| Chlorure de sodium | 2 % en poids |

### Méthode de fabrication

La phase grasse A (contenant les charges) et la phase aqueuse B sont pesées séparément et mélangées à l'aide d'une spatule.

Puis la phase aqueuse est introduite dans la phase grasse, sous ancre, en plusieurs fractions ; l'agitation est maintenue pendant environ 10 minutes puis l'émulsion est passée dans un homogénéisateur à filière (ALM™ filière A180). Les observations et analyses des émulsions sont consignées dans le tableau suivant :

| Poudre | Poudre (t) | | Poudre (3) | |
|---|---|---|---|---|
| x (% en poids) | 2% | 5% | 2% | 5% |
| Viscosité (Brookfield LVT) Mobile 4 Vitesse 6 | 20000 mPa.s | 8500 mPa.s | 23300 mPa.s | 9260 mPa.s |
| Stabilité à 25°C | Exsudation huileuse à 3 mois | Exsudation huileuse à 3 mois | Stable après 3 mois | Stable après 3 mois |
| Stabilité à 40 °C | Exsudation huileuse à 1 mois | Exsudation huileuse à 3 mois | Stable après 3 mois | Stable après 3 mois |
| Stabilité à 50° C | Exsudation huileuse à 1 mois | Exsudation huileuse à 3 mois | stable après 1 mois | stable après 3 mois |
| Toucher | Etalement facile Toucher doux Léger effet poudré | Etalement facile Toucher très doux Effet poudré rémanent | Etalement facile Toucher doux Léger effet poudré | Etalement facile Toucher très doux Effet poudré rémanent |

Tout en conservant les propriétés sensorielles de la poudre choisie initialement, les poudres homogènes selon l'invention permettent d'améliorer significativement, et sans modification du procédé de fabrication, la stabilité des émulsions réalisées, même pour un faible pourcentage de poudre.

### (4) - Amélioration des propriétés sensorielles des poudres homogènes selon l'invention par rapport à celles des agents de texture en poudre classiques

On a préparé plusieurs poudres homogènes selon l'invention à partir de différents agents de textures en poudre en mettant en oeuvre le procédé décrit au paragraphe (1) précédent.

Les qualités sensorielles des poudres homogènes, en tant que matières premières, sont appréciées par un panel de 15 experts qui notent chaque critère évalué :

### Qualité du toucher entre le pouce et l' index (critère 1)

Echelle de notes : de 0 à 5 (Toucher rêche : 0 ; Toucher très doux : 5) ; Pouvoir couvrant (critère 2)

Echelle de notes : de -1 à 1 (Pouvoir couvrant identique à celui de la poudre témoin : 0 ; Pouvoir couvrant supérieur à celui de la poudre témoin : 1 ; Pouvoir couvrant inférieur à celui de la poudre de départ : -1) ;

### Adhérence sur la peau (critère 3)

Echelle de notes : de 0 à 5 (Pas d'adhérence : 0; très adhérente : 5)

On a comparé les propriétés des couples de poudres suivants :
Poudre témoin (tₐ) : MICROPEARL™ M100
Poudre homogène selon l'invention (4ₐ) : MICROPEARL™ M100 + SIMULGEL™ NS (rapport pondéral 80 / 20)
Poudre témoin (t_{b}) : AEROSIL™ 200
Poudre homogène selon l'invention (4_{b}) :AEROSIL™ 200 + SIMULGEL™ NS : (rapport pondéral 80 / 20)
Poudre témoin (t_{c}) : Mica 1000™
Poudre homogène selon l'invention (4_{c}) : MICA 1000™ + SIMULGEL™ NS (rapport pondéral 80 / 20)
Poudre témoin (t_{d}) : DRY FLO™
Poudre homogène selon l'invention (4_{d}) : DRY FLO™ + SIMULGEL™ NS (rapport pondéral 80 / 20)
Poudre témoin (tₑ) : Oxyde de Titane USP
Poudre homogène selon l'invention (4ₑ) : Oxyde de Titane USP + SIMULGEL™ NS (rapport pondéral 80 / 20)
Poudre témoin (t_{f}) : Oxyde de Zinc micronisé : ZnO Neutral™
Poudre homogène selon l'invention (4_{f}) : Oxyde de Zinc Neutral + SIMULGEL™ NS (rapport pondéral 80 / 20)

Les résultats, consignés dans le tableau suivant, sont les moyennes arithmétiques des notes pour chacun des trois critères.

| | Compositions | | | | | | | | | | | |
|---|---|---|---|---|---|---|---|---|---|---|---|---|
| | (tₐ) | (4ₐ) | (t_{b}) | (4_{b}) | (t_{c}) | (4_{c}) | (t_{d}) | (4_{d}) | (tₑ) | (4ₑ) | (tₑ) | (4_{f}) |
| Critère 1 | 3,5 | 4,7 | 1,2 | 2,5 | 3,0 | 4,1 | 3,2 | 4,3 | 0,5 | 1,5 | 0,7 | 1,4 |
| Critère 2 | 0 | 0 | 0 | 0 | 0 | 1 | 0 | 0 | 0 | 0 | 0 | 0 |
| Critère 3 | 2,5 | 4,4 | 1,9 | 2,8 | 3,3 | 4,1 | 2,2 | 3,6 | 3,7 | 4,9 | 4,0 | 4,7 |

De façon générale, quelle que soit la nature de la poudre cosmétiquement ou pharmaceutiquement acceptable choisie et ses propriétés intrinsèques, son toucher initial doux ou rêche, sa forte ou faible adhérence naturelle sur la peau, les poudres homogènes selon l'invention ont un meilleur score au toucher et à l'adhérence sur la peau que les poudres témoins.

Dans la majorité des cas, le pouvoir couvrant des poudres homogènes selon l'invention est le même que celui de la poudre témoin correspondante.

Les poudres transparentes comme les polyméthacrylate de méthyle (MICROPEARL), les silices ou les dérivés d'amidon donnent des compositions transparentes tandis que les compositions fabriquées à partir de poudres très couvrantes telles que les oxydes de titane ou de zinc, ont le même pouvoir couvrant de la poudre d'origine.

### (5) - Evaluation des propriétés sensorielles en formulation

On a préparé des poudres pressées à partir de la formule suivante :

### Formule de poudre pressée

### Formule

| | |
|---|---|
| Talc LUZENAC™ 00C | QSP 100 % |
| (composition selon l'invention) | 05,00 % en poids |
| Mica 1000™ | 50, 00 % en poids |
| Colorant "FDC Yellow n°6 A1 lake" | 00,30 % en poids |
| Colorant "Ariabel Sienna" | 00,20 % en poids |
| LANOL™ 1688 | 05,00 % en poids |
| Diméthicone | 05,00 % en poids |

Les composés pulvérulents sont mélangés puis broyés avec un mélangeur à couteaux. Les liants hydrophobes sont ensuite ajoutés l'un après l'autre avec un broyage du mélange entre chaque ajout. Le mélange final est encore broyé pendant quelques secondes. La poudre est ensuite compactée dans une coupelle métallique au moyen d'une compacteuse manuelle KEMWALL™ sous une pression de 80 10⁵ Pa.

Les qualités sensorielles des formulations sont appréciées par un panel de 15 individus qui notent sur une échelle de 0 à 5 les critères suivants :
Facilité de prélèvement au doigt (critère 1)
   Echelle de notes de 0 à 5 (prélèvement nul : 0; 5 = prélèvement très facile :5)
Facilité d'étalement sur la peau (critère 2)
   Echelle de notes de 0 à 5 (étalement difficile, accroche :; étalement très facile : 5)
Relargage du colorant, homogénéité du film, répartition de la couleur (critère 3)
   Echelle de notes de 0 à 5 (film hétérogène, mauvaise couverture de la peau, colorant mal réparti : 0; film parfaitement homogène, couleur uniforme :0)
Toucher sur la peau (critère 4)
   Echelle de notes de 0 à 5 (toucher rêche : 0 ; toucher très doux : 5)

On a comparé les propriétés des formulations suivantes :
Formulation (t) : MICROPEARL™ M310
Formulation selon l'invention (5) : MICROPEARL™ M100 + SIMULGEL™ EG (rapport pondéral 60 / 40).

Les résultats, consignés dans le tableau suivant, sont les moyennes arithmétiques des notes pour chacun des quatre critères.

| | Formulations | |
|---|---|---|
| | Formulation (t) | Formulation (5) |
| Aspect visuel des compacts | Aspect lisse et homogène | Aspect lisse et homogène |
| Critère 1 | 3,0 | 4,2 |
| Critère 2 | 3,5 | 3,8 |
| Critère 3 | 4,0 | 4,5 |
| Critère 4 | 3.2 | 4,7 |

Les poudres homogènes selon l'invention sont également des agents de texture remarquables dans les formules de poudres libres, pressées ou coulées.

### (6) - Effet stabilisant au sein d'une émulsion eau dans silicone

On a comparé les propriétés de la série d'émulsions préparées au paragraphes (3) précédent à celles de la série d'émulsions dans des huiles de silicone sur la base de la formule suivante :

| Phase A : | |
|---|---|
| DC5225C™ | 20 % en poids |
| DC345™ | 10 % en poids |
| Sepicide™ HB | 0,3 % en poids |
| MICROPEARL™ M310 | (0,8 x) % en poids |

| Phase B | |
|---|---|
| SIMULGEL™ EG | (0,2 x) % en poids |

| Phase C | |
|---|---|
| Eau | qsp 100% |
| Sepicide™ CI | 0,2 % en poids |
| Glycérine | 5 % en poids |
| Chlorure de sodium | 2 % en poids |

### Méthode de fabrication

La phase grasse A (contenant les charges) et la phase aqueuse B sont pesées séparément et mélangées à l'aide d'une spatule.

La phase aqueuse C est introduite dans la phase grasse, sous ancre, en plusieurs fractions ; l'agitation est maintenue quelques minutes, le SIMULGEL™ EG est introduit dans le mélange, l'agitation poursuivie pendant environ 10 minutes puis l'émulsion est passée dans un homogénéisateur à filière (ALM™ filière A180). Les observations et analyses des émulsions sont consignées dans le tableau suivant :

| | Emulsions selon l'état de la technique | | Emulsions selon l'invention | |
|---|---|---|---|---|
| x | 2 | 5 | 2 | 5 |
| Viscosité (Brookfield LVT) Mobile 4 Vitesse 6 | 21000 mPa.s | 8800 mPa.s | 23300 mPa.s | 9260 mPa.s |
| Stabilité à 25°C | Exsudation huileuse à 3 mois | Stable après 3 mois | Stable après 3 mois | Stable après 3 mois |
| Stabilité à 40 °C | Exsudation huileuse à 3 mois | Exsudation huileuse à 3 mois | Stable après 3 mois | Stable après 3 mois |
| Stabilité à 50° C | Exsudation huileuse à 1 mois | Exsudation huileuse à 3 mois | stable après 1 mois | stable après 3 mois |
| Toucher | Etalement facile ; Toucher doux ; Léger effet poudré | | Etalement facile Toucher doux Léger effet poudré | Etalement facile Toucher très doux Effet poudré rémanent |

L'utilisation des poudres homogènes selon l'invention permet d'améliorer la tenue à la température des émulsions d'huiles de silicone sans nuire à leurs propriétés sensorielles.

### B) Exemples de formulations cosmétiques

Les poudres homogènes selon l'invention sont des agents de texture polyvalents, aussi performants dans les milieux liquides aqueux ou huileux que dans les formulations solides moulées ou poudres.

### Exemple 1 : Lotion purifiante pour peaux grasses

### Formule

| | | |
|---|---|---|
| Phase A | Eau | QSP 100 % |
| | Gluconate de Cuivre | 0,05 % |
| | Gluconate de Zinc | 0,15 % |
| Phase B | MICROPEARL™ M310 + SIMULGEL™ EG (75 / 25 en poids) | 3,50 % |
| Phase C | SEPICIDE™ HB | 0,30 % |
| | SEPICIDE™ LD | 0,80 % |
| | Parfum | 0,10% |

### Méthode

La phase A est préparée en dispersant sous agitation le composé pulvérulent dans l'eau puis les phases B et C sont ajoutées au gel en maintenant l'agitation.

### Exemple 2 - Fluide poudré pour imprégnation sur lingettes

### Formule

| | | |
|---|---|---|
| Phase A | Eau | QSP 100 % |
| | Glycérine | 3,00 % |
| | MICROPEARL™ M310 + SIMULGEL™ EG | 2,4 % |
| | (60 / 40 en poids) (poudre homogène selon l'invention) | |
| Phase B | SEPICIDE™ HB | 0,30 % |
| | SEPICIDE™ LD | 0,80 % |
| | Parfum | 0,10% |

### Méthode

La phase A est préparée en dispersant sous agitation le composé pulvérulent dans l'eau puis les phases B et C sont ajoutées au gel en maintenant l'agitation.

### Exemple 3 : Fluide vaporisable douceur

### Formule

| | | |
|---|---|---|
| Phase A | Eau | QSP 100 % |
| | MICROPEARL™ M201 + SIMULGEL™ EG | 5,00 % |
| | (80 / 20 en poids) (poudre homogène selon l'invention) | |
| Phase B | DC345™ | 2,00 % |
| Phase C | SEPICIDE™ HB | 0,30 % |
| | SEPICIDE™ CI | 0,20 % |
| | Parfum | 0,15 % |
| | SENSIVA™ SC50 | 0,50 % |

### Méthode

La phase A est préparée en dispersant sous agitation la composition selon l'invention dans l'eau puis les phases B et C sont ajoutées au gel en maintenant l'agitation.

### Exemple 4 - Gel rafraîchissant après-soleil

### Formule

| | | |
|---|---|---|
| Phase A | Alcool à 90° | 20,00 % |
| | Menthol | 00,05 % |
| Phase B | Aqua/Water | QSP 100% |
| | MICROPEARL™ M201 + SIMULGEL™ EG | 10,00 % |
| | (80 / 20 en poids) (poudre homogène selon l'invention) | |
| Phase C | SEPICALM™ VG | 03,00 % |
| | Parfum | 00,10 % |
| | Colorant | QS |

### Méthode

La phase A est préparée en solubilisant le menthol dans l'éthanol.

La phase B est préparée en dispersant la poudre homogène selon l'invention dans l'eau sous agitation puis, quand le gel est homogène, la phase C puis la phase A sont ajoutées sur la phase B.

### Exemple 5 : Soin corporel raffermissant

### Formule

| | | |
|---|---|---|
| Phase A | Eau | QSP 100 % |
| | MICROPEARL™ 305 + SIMULGEL™ EG | 8,50 % |
| | (80 / 20 en poids) (poudre homogène selon l'invention) | |
| Phase B | LANOL™ 99 | 5,00 % |
| | SEPICALM™ VG | 1,00 % |
| | SEPILIFT™ DPHP | 1,00 % |
| Phase C | SEPICIDE™ HB | 0,30% |
| | SEPICIDE™ CI | 0,20 % |
| | Parfum | 0,10 % |

### Méthode

La poudre homogène selon l'invention est dispersée dans l'eau sous agitation.

La phase B est préparée en chauffant l'ester à 70°C, puis en ajoutant le SEPICALM™ VG et le SEPILIFT™ DPHP.

Cette phase B est ajoutée sous agitation à la phase A, puis la phase C est également ajoutée dans le mélange ainsi formé.

### Exemple 6 : Soin dynamisant

### Formule

| | | |
|---|---|---|
| Phase A | Eau | QSP 100% |
| | Glycérine | 02,50 % |
| | MICROPEARL™ 310 + SIMULGEL™ EG | 13,00 % |
| | (80/20 en poids) (poudre homogène selon l'invention) | |
| | SEPITONIC™ M3 | 01,00 % |
| Phase B | LANOL™ 99 | 05,00% |
| | DC345™ | 02,50 % |
| Phase C | Parfum | 00,10 % |
| | SEPICIDE™ HB | 00,30 % |
| | SEPICIDE™ CI | 00,20 % |

### Méthode

Disperser le composé pulvérulent sous agitation dans la phase aqueuse puis introduire la phase grasse B dans la phase aqueuse A en maintenant l'agitation. Ajouter la phase C dans le gel final.

### Exemple 7 : Rouge à lèvres

### Formule:

| | | |
|---|---|---|
| | Oléate de décyle | 25,00 % |
| | Dioxyde de titane | 6,44 % |
| | Oxyde de fer jaune | 3,04 % |
| Phase A | Oxyde de fer noir | 0,36 % |
| | Colorant "DC RED 7" | 0,78 % |
| | Colorant "FDC YELLOW 6" | 0,70 % |
| | Colorant "FDC BLUE 1" | 0,17 % |
| | LANOL™ 99 | Qsp 100 % |
| | Ozokérite | 11,75 % |
| | Ricinoléate de cétyle | 10,00 % |
| Phase B | Octydodécanol | 8,12 % |
| | Cire d'abeille | 4,20 % |
| | Trilinoléate de Tri-isostéaryle | 5,00 % |
| | Palmitate de cétyle | 4,50 % |
| | Cire de carnauba (Copernicia cerifera) | 2,28 % |
| | SEPILIFT™ DPHP | 1,00 % |
| | MICROPEARL™ MHB + SIMULGEL™ NS (65 / 35 en poids) | 3,00 % |
| Phase C | Parfum | 1,25 % |
| | Acétate de Tocophéryle | 00,20 % |

### Méthode

Broyer la phase A au broyeur à billes.

Faire fondre la phase B à 85°C-90°C puis ajouter, sous agitation, la phase A préalablement broyée. Agiter jusqu'à complète dispersion.

Introduire la phase C sous agitation. Couler la pâte à chaud dans les moules.

### Exemple 8 : Poudre pour le visage

### Formule

| | | |
|---|---|---|
| | GIVOBIO™ GCu | 0,50 % |
| | LIPACIDE™ C8G | 0,50 % |
| | LIPACIDE UG | 0,50 % |
| Phase A | MICROPEARL MHB™ + SEPIGEL™ 305 | 5,00 % |
| | (60% + 40%) (poudre homogène selon l'invention) | |
| | Mica | 50,00 % |
| | Talc | 33,00 % |
| | Colorant "FDC Yellow 6 lake" | 0,30 % |
| | Colorant "Ariabel Sienna" | 0,20 % |
| Phase B | LANOL™ 99 | 5,00 % |
| Phase C | Diméthicone | 5,00 % |

### Méthode

Peser toutes les poudres (phase A) et les broyer à sec dans un broyeur à couteaux.

Ajouter la phase B et reproduire le même temps de broyage que pour la phase A.

Ajouter la phase C et répéter la même opération de broyage que pour la phase B.

La poudre ainsi préparée est ensuite pressée dans des godets au moyen d'une compacteuse manuelle KENWALL™ sous une pression de 80 10⁵ Pa.

### Exemple 9 : Fond de teint

### Formule

| | | |
|---|---|---|
| Phase A | Eau | 9,50 % |
| | Butylène glycol | 2,00 % |
| | PEG-400 | 2,00 % |
| | PECOSIL™ PS100 | 0,50 % |
| | Hydroxyde de sodium | QS pH=9 |
| | Dioxyde de titane | 3,50 % |
| | Talc | 1,00 % |
| | Oxyde de fer jaune | 0,41 % |
| | Oxyde de fer rouge | 0,15 % |
| | Oxyde de fer noir | 0,025 % |
| Phase B | MONTANOV™ L | 2,00 % |
| | LANOL™ 99 | 4,00 % |
| | Caprylic capric triglycéride | 4,00 % |
| Phase C | DC345™ | 2,00 % |
| | Gomme de xanthane | 0,30 % |
| | Silicate de magnésium et d'aluminium | 1,00 % |
| Phase D | Eau | QSP 100% |
| | Tetrasodium EDTA | 0,05 % |
| | MICROPEARL™ M305 + SIMULGEL™ NS | 2,00 % |
| | (80%/ 20%) (poudre homogène selon l'invention) | |
| Phase E | SEPICIDE™ HB | 0,50 % |
| | SEPICIDE™ CI | 0,30 % |
| | Parfum | 0,20 % |

### Méthode

Les composés liquides de la phase A sont mélangés puis le pH est ajusté avant l'ajout des pigments ; cette phase pigmentaire est broyée avec un broyeur à billes.

La phase B est ensuite fondue à 75°C.

L'eau est également portée à 75°C avant l'ajout de la phase D puis de la phase A.

Puis la phase C est ajoutée dans la phase B, et ce mélange est introduit dans la phase aqueuse chaude avant le déclenchement de l'émulseur.

L'émulsion est ensuite progressivement refroidie et les constituants de la phase E sont ajoutés à 30°C.

### Exemple 10 : Gel-crème teinté

### Formule

| | | |
|---|---|---|
| Phase A | Eau | 10,00% |
| | Butylène glycol | 4,00 % |
| | PEG-400 | 4,00 % |
| | PECOSIL™ PS100 | 1,50 % |
| | Hydoxyde de sodium | qs pH=7 |
| | Dioxyde de Titane | 2,00 % |
| | Oxyde de fer jaune | 0,80 % |
| | Oxyde de fer rouge | 0,30 % |
| | Oxyde de fer noir | 0,05 % |
| Phase B | LANOL™ 99 | 4,00 % |
| | Caprylic capric triglyceride | 4,00 % |
| | DC345™ | 4,00 % |
| | SEPICIDE™ HB | 0,30 % |
| | Parfum | 0,20 % |
| Phase C | Eau | QSP 100% |
| | Tetrasodium EDTA | 0,05 % |
| | SEPICONTROL™ A5 | 4,00 % |
| | SEPICIDE CI (lmidazolidinyl urea - SEPPIC) | 0,20 % |
| Phase D | MICROPEARL™ M100 + SEPIGEL™ 305 (80%/ 20%) (composition selon l'invention) | 17,5 % |

### Méthode :

Les composés liquides de la phase A sont mélangés avant l'ajout des pigments puis cette phase pigmentaire A est broyée avec un broyeur à billes.

La phase D est introduite sous agitation turbulente dans la phase C. Quand le gel est formé et homogène, la phase grasse B est ajoutée puis, en dernier, la pâte pigmentaire A.

### Exemple 11 : Emulsion solaire de type Eau - Silicone

### Formule :

| | | |
|---|---|---|
| Phase A | DC5225C | 20,00 % |
| | DC345™ | 10,00 % |
| | SEPICALM™ VG | 3,00 % |
| | Dioxyde de titane | 5,00 % |
| | Oxyde de Zinc Z-COTE ™ + SIMULGEL NS™ | 5,00 % |
| | (80%/ 20%) (poudre homogène selon l'invention) | |
| | SEPICIDE HB™ | 0,30 % |
| | Parfum | 0,05 % |
| Phase B | Eau | QSP 100% |
| | SEPICIDE™ CI | 0,20 % |
| | Glycérine | 5,00 % |
| | Chlorure de sodium | 2,00 % |

### Méthode :

La phase A est préparée en mélangeant les silicones et le SEPICALM™ VG puis en dispersant les charges minérales sous agitation douce jusqu'à leur mouillage complet puis en ajoutant le conservateur et le parfum.

La phase aqueuse B est préparée séparément puis est introduite lentement dans la phase A sous agitation modérée. L'étape d'homogénéisation commence après l'introduction de la totalité des composants.
En milieu aqueux continu, les poudres homogènes selon l'invention sont particulièrement performants car ils montrent, outre leurs qualités sensorielles, des propriétés de mise en suspension et un effet viscosant intrinsèque, modulable en fonction de la dose d'utilisation.
La simple dispersion aqueuse des poudres homogènes selon l'invention, sans ajout d'autre ingrédient, aboutit à une suspension de poudre stable et de viscosité modulable en fonction de la dose de poudre introduite.
Cette suspension peut également stabiliser, en fonction de la dose de poudre, des huiles de toute nature et des solvants couramment utilisés en cosmétique tels que l'éthanol, les glycols comme le propylène glycol, butylène glycol ou l'hexylène glycol.
En milieu liquide continu huile, un effet stabilisant notable est également apporté par les poudres homogènes de l'invention, en fonction de la nature de la poudre choisie.
Les poudres homogènes de l'invention sont des agents de texture performants dans tous les types de formulation, ils apportent :
Un toucher très doux, amélioré par rapport aux agents de texture poudre utilisés seuls
Une excellente adhérence sur la peau, supérieure aux agents de texture poudre utilisés seuls.

Les caractéristiques des produits commerciaux utilisés dans les exemples précédents, sont les suivantes :
MICROPEARL™ M305 : poudre hydrodispersible soyeuse à base de polyméthylméthacrylate réticulé
MICROPEARL™ M310 : poudre hydrophobe soyeuse à base de polyméthylméthacrylate réticulé
MICROPEARL™ M 100 : poudre hydrodispersible soyeuse à base de polyméthylméthacrylate ;
MICROPEARL™ M201 : poudre hydrodispersible soyeuse à base de polyméthylméthacrylate réticulé de granulométrie environ 1 à 5µm
MICROPEARL™ MHB : poudre hydrophobe soyeuse à base de polyméthylméthacrylate réticulé ;
SIMULGEL™ EG : latex inverse auto-inversible de copolymère tel que ceux décrits dans la publication internationale WO 99/36445 (dénomination INCI : Sodium acrylate/Sodium acryloyldimethyl taurate copolymer et Isohexadecane et Polysorbate 80) commercialisé par la société SEPPIC ;
SIMULGEL™ NS : latex inverse auto-inversible de copolymère tel que ceux décrits dans la publication internationale WO 99/36445 (dénomination INCI : hydroxyethylacrylate/Sodium acryloyldimethyl taurate copolymer et squalane et Polysorbate 60) commercialisé par la société SEPPIC ;
SEPIGEL™ 305 : latex inverse auto-inversible (dénomination INCI : Polacrylamide / C13-14 Isoparaffin / Laureth-7)
DC5225C™ : Mélange de cyclopentasiloxane and de diméthicone copolyol commercialisé par la société DOW CORNING ;
DC345™ : cyclométhicone commercialisé par la société DOW CORNING ;
DRY FLO™ : Amidon modifié avec de l'aluminium et du succinate d'octènyle commercialisé par la société NATIONAL STARCH ;
Mica 1000 ™ : poudre de mica commercialisé par la société SCIAMA ;
AEROSIL™ 200 : Silice commercialisée par la société DEGUSSA ; ZnO Neutral™ : Oxyde de Zinc micronisé commercialisé par la société HARMANN & REIMER ;
SEPICIDE™ CI : imidazoline urée, (agent conservateur) commercialisé par la société SEPPIC ;
SEPICIDE™ HB : Mélange de phénoxyéthanol, de méthylparaben, d'éthylparaben, de propyl-paraben et de butylparaben, (agent conservateur) commercialisé par la société SEPPIC ;
SEPICIDE™ LD : phénoxyéthanol commercialisé par la société SEPPIC ;
SENSIVA™ SC50 : 1-(2-éthyl hexyl) glycérol commercialisé par la société SCHUELKE & MAYR ;
SEPICALM™ VG : composition telle que celles décrites dans la publication internationale WO 99/45899 (Dénomination INCI : sodium palmitoyl proline et extrait de fleur de nénuphar), commercialisé par la société SEPPIC ;
SEPILIFT™ DPHP : (Dénomination INCI : Dipalmitoyl hydroxyproline), commercialisé par la société SEPPIC ;
SEPITONIC™ M3 : mélange d'aspartate de magnésium, de gluconate de cuivre et de gluconate de zinc commercialisé par la société SEPPIC ;
GIVOBIO™ GCu : gluconate de cuivre commercialisé par la société SEPPIC ;
LIPACIDE™ UG : undecylenoylglycine commercialisé par la société SEPPIC ;
LIPACIDE™ C8G : octanoylglycine commercialisé par la société SEPPIC ;
LANOL™ 99 : isononanoate d'isononyle commercialisé par la société SEPPIC ;
LANOL™ 1688 : éthylhexanoate de cétéaryle commercialisé par la société SEPPIC ;
Le PECOSIL™ PS 100 est du diméthicone copolyol phosphate commercialisé par la société PHOENIX ;
MONTANOV™ L : agent émulsionnant à base d'alcool en C14-C22 et d'alkyl polyglucoside en C12-C20 tel que ceux décrits dans la demande de brevet européen EP 0 995 487 ;
SEPICONTROL™ A5 : mélange de capryloyl glycine, de sarcosine et d'extrait de cinnamon zylanicum, commercialisé par la société SEPPIC, tel que ceux décrits dans la publication internationale WO 99/00109 ;

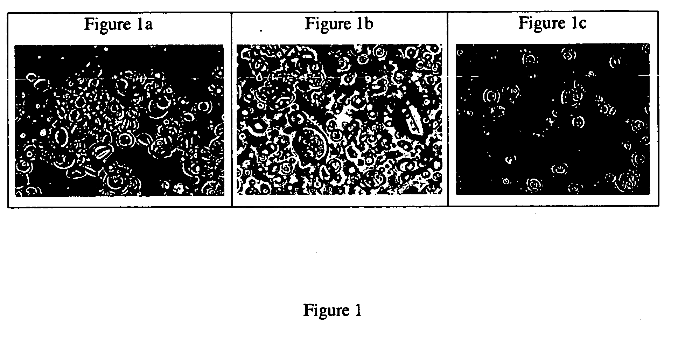

## Revendications

1. Poudre homogène consistant essentiellement en un mélange contenant :
- de 5% à 80 % en poids d'au moins une composition comprenant une phase huile, une phase aqueuse, au moins un agent émulsionnant de type eau dans huile (E/H), au moins un agent émulsionnant de type huile dans eau (H/E), comprenant de 20% à 70% en poids, et de préférence de 25% à 50% en poids, d'un polyélectrolyte branché ou réticulé, **caractérisé en ce que** ledit polyélectrolyte est, soit un homopolymère à base d'un monomère possédant ou bien une fonction acide fort partiellement ou totalement salifiée, ou bien une fonction acide faible partiellement ou totalement salifiée, soit un copolymère à base d'au moins un monomère possédant une fonction acide fort, copolymérisé ou bien avec au moins un monomère possédant une fonction acide faible ou bien avec au moins un monomère neutre, soit un copolymère à base d'au moins un monomère possédant une fonction acide faible, copolymérisé avec au moins soit un monomère neutre, soit un monomère possédant une fonction acide faible;
- de 95 % à 20% en poids du mélange d'au moins une poudre cosmétiquement ou pharmaceutiquement acceptable.

2. Poudre homogène telle que définie à la revendication 1, dans laquelle le polyélectrolyte présent dans la composition telle que définie précédemment, est choisi parmi les polyéléctrolytes suivants:
copolymère d'acide acrylique partiellement salifié sous forme de sel de sodium et d'acrylamide, réticulé au méthylène bis(acrylamide) ;
copolymère d'acide 2-méthyl 2-[(1-oxo 2-propènyl) amino] 1-propanesulfonique partiellement salifié sous forme de sel de sodium et d'acrylamide, réticulé au méthylène bis(acrylamide) ;
copolymère d'acide 2-méthyl 2-[(1-oxo 2-propènyl) amino] 1-propanesulfonique partiellement salifié sous forme de sel de sodium et d'acide acrylique partiellement salifié sous forme de sel de sodium, réticulé au méthylène bis(acrylamide) ;
copolymère d'acide 2-méthyl 2-[(1-oxo 2-propènyl) amino] 1-propanesulfonique partiellement salifié sous forme de sel de sodium et d'acrylate de 2-hydroxy éthyle, réticulé au méthylène bis(acrylamide) ;
homopolymère d'acide 2-méthyl 2-[(1-oxo 2-propènyl) amino] 1-propanesulfonique partiellement salifié sous forme de sel de sodium réticulé au méthylène bis(acrylamide) ;
homopolymère d'acide acrylique partiellement salifié sous forme de sel d'ammonium ou de sel de monoéthanol amine, réticulé au diallyloxyacétate de sodium ou
homopolymère d'acide acrylique partiellement salifié sous forme de sel d'ammonium ou de sel de monoéthanol amine, réticulé au triallyl amine.

3. Poudre homogène telle que définie à l'une des revendications 1 ou 2, dans laquelle la poudre cosmétiquement ou pharmaceutiquement acceptable est constituée de microsphères poreuses polyméthylméthacrylate, de surface spécifique supérieure ou égale à 0,5 m² par gramme.

4. Poudre homogène telle que définie à l'une des revendications 1 à 3, **caractérisée en ce qu'**elle contient au moins 50 % en poids de poudre cosmétiquement ou pharmaceutiquement acceptable.

5. Utilisation de la poudre homogène telle que définie à l'une des revendications 1 à 4, comme agent de texture des formulations, cosmétiques ou pharmaceutiques.

6. Utilisation de la poudre homogène telle que définie à l'une des revendications 1 à 4 dans les formulations solides et plus particulièrement dans les fonds de teint, les poudres de maquillage, les mascaras ou les rouges à lèvres.

7. Utilisation de la poudre homogène telle que définie à l'une des revendications 1 à 6 dans les formulations liquides et plus particulièrement dans les d'émulsions, les lotions ou les gels et tout particulièrement dans des formulations vaporisables ou encore dans des solutions imprégnées sur tissus ou papier et plus particulièrement sur des lingettes ou sur des papiers correcteurs de teint.

## Claims

1. Homogeneous powder consisting essentially of a mixture containing:
- from 5% to 80% by weight of at least one composition comprising an oil phase, an aqueous phase, at least one water-in-oil (W/O) emulsifier, at least one oil-in-water (O/W) emulsifier, containing from 20% to 70% by weight and preferably from 25% to 50% by weight of a branched or crosslinked polyelectrolyte, **characterized in that** the said polyelectrolyte is alternatively a homopolymer based on a monomer possessing either a strong acid function which is partly or totally in salt form or a weak acid function which is partly or totally in salt form, or a copolymer based on at least one monomer possessing a strong acid function copolymerized either with at least one monomer possessing a weak acid function or with at least one neutral monomer, or a copolymer based on at least one monomer possessing a weak acid function copolymerized with at least one neutral monomer or with a monomer possessing a weak acid function;
- from 95% to 20% by weight of at least one cosmetically or pharmaceutically acceptable powder.

2. Homogeneous powder as defined in Claim 1, wherein the polyelectrolyte present in the composition as defined above is selected from the following polyelectrolytes:
copolymer of acrylic acid partly in sodium salt form and acrylamide, crosslinked with methylenebis-(acrylamide);
copolymer of 2-methyl-2-[(1-oxo-2-propenyl)-amino]-1-propanesulphonic acid partly in sodium salt form and acrylamide, crosslinked with methylenebis-(acrylamide);
copolymer of 2-methyl-2-[(1-oxo-2-propenyl)-amino]-1-propanesulphonic acid partly in sodium salt form and acrylic acid partly in sodium salt form, crosslinked with methylenebis(acrylamide);
copolymer of 2-methyl-2-[(1-oxo-2-propenyl)-amino]-1-propanesulphonic acid partly in sodium salt form and 2-hydroxyethyl acrylate, crosslinked with methylenebis(acrylamide);
homopolymer of 2-methyl-2-[(1-oxo-2-propenyl)-amino]-1-propanesulphonic acid partly in sodium salt form, crosslinked with methylenebis(acrylamide);
homopolymer of acrylic acid partly in ammonium salt or monoethanolamine salt form, crosslinked with sodium diallyloxyacetate; or
homopolymer of acrylic acid partly in ammonium or monoethanolamine salt form, crosslinked with triallylamine.

3. Homogeneous powder as defined in either of Claims 1 and 2, wherein the cosmetically or pharmaceutically acceptable powder is composed of porous polymethyl methacrylate microspheres having a specific surface area greater than or equal to 0.5 m² per gram.

4. Homogeneous powder as defined in any one of Claims 1 to 3, **characterized in that** it contains at least 50% by weight of cosmetically or pharmaceutically acceptable powder.

5. Use of the homogeneous powder as defined in any one of Claims 1 to 4 as a texturizer in cosmetic or pharmaceutical formulations.

6. Use of the homogeneous powder as defined in any one of Claims 1 to 4 in solid formulations and more particularly in foundations, makeup powders, mascaras or lipsticks.

7. Use of the homogeneous powder as defined in any one of Claims 1 to 6 in liquid formulations and more particularly in emulsions, lotions or gels and very particularly in sprayable formulations or else in solutions impregnated on fabrics or paper and more particularly on towelettes or on complexion corrector papers.

## Patentansprüche

1. Homogenes Pulver, das im wesentlichen aus einer Mischung besteht, die folgendes enthält:
- 5 Gew.-% bis 80 Gew.-% einer Zusammensetzung, umfassend eine Ölphase, eine wäßrige Phase, mindestens einen Emulgator des Wasser-in-Öl (W/O)-Typs, mindestens einen Emulgator des Ölin-Wasser (O/W)-Typs, umfassend 20 Gew.-% bis 70 Gew.-%, vorzugsweise 25 Gew.-% bis 50 Gew.-%, eines verzweigten oder vernetzten Polyelektrolyten, **dadurch gekennzeichnet, daß** es sich bei dem Polyelektrolyten entweder um ein Homopolymer auf Grundlage eines Monomers mit entweder einer teilweise oder ganz versalzten starken Säurefunktion oder einer teilweise oder ganz versalzten schwachen Säurefunktion oder um ein Copolymer auf Grundlage von mindestens einem Monomer mit einer starken Säurefunktion, das entweder mit mindestens einem Monomer mit einer schwachen Säurefunktion oder mit mindestens einem neutralen Monomer copolymerisiert ist, oder um ein Copolymer auf Grundlage von mindestens einem Monomer mit einer schwachen Säurefunktion, das mit mindestens entweder einem neutralen Monomer oder einem Monomer mit einer schwachen Säurefunktion copolymerisiert ist, handelt;
- 95 Gew.-% bis 20 Gew.-% der Mischung von mindestens einem kosmetisch oder pharmazeutisch unbedenklichen Pulver.

2. Homogenes Pulver nach Anspruch 1, in dem der in der wie zuvor definierten Zusammensetzung vorliegende Polyelektrolyt aus der Reihe der folgenden Polyelektrolyte stammt:
Copolymer aus teilweise in Form des Natriumsalzes versalzter Acrylsäure und Acrylamid, das mit Methylenbis(acrylamid) vernetzt ist;
Copolymer aus teilweise in Form des Natriumsalzes versalzter 2-Methyl-2-[(1-oxo-2-propenyl)amino]-1-propansulfonsäure und Acrylamid, das mit Methylenbis(acrylamid) vernetzt ist;
Copolymer aus teilweise in Form des Natriumsalzes versalzter 2-Methyl-2-[(1-oxo-2-propenyl)amino]-1-propansulfonsäure und teilweise in Form des Natriumsalzes versalzter Acrylsäure, das mit Methylenbis (acrylamid) vernetzt ist;
Copolymer aus teilweise in Form des Natriumsalzes versalzter 2-Methyl-2-[(1-oxo-2-propenyl)amino]-1-propansulfonsäure und 2-Hydroxyethylacrylat, das mit Methylenbis(acrylamid) vernetzt ist;
Homopolymer aus teilweise in Form des Natriumsalzes versalzter 2-Methyl-2-[(1-oxo-2-propenyl)amino]-1-propansulfonsäure, das mit Methylenbis(acrylamid) vernetzt ist;
Homopolymer aus teilweise in Form des Ammoniumsalzes oder des Monoethanolaminsalzes versalzter Acrylsäure, das mit Natriumdiallyloxyacetat vernetzt ist, oder
Homopolymer aus teilweise in Form des Ammonium- oder Monoethanolaminsalzes versalzter Acrylsäure, das mit Triallylamin vernetzt ist.

3. Homogenes Pulver nach Anspruch 1 oder 2, in dem das kosmetisch oder pharmazeutisch unbedenkliche Pulver aus porösen Polymethylmethacrylat-Mikrosphären mit einer spezifischen Oberfläche von 0,5 m² oder mehr pro Gramm besteht.

4. Homogenes Pulver nach einem der Ansprüche 1 bis 3, **dadurch gekennzeichnet, daß** es mindestens 50 Gew.-% kosmetisch oder pharmazeutisch unbedenkliches Pulver enthält.

5. Verwendung des homogenen Pulvers nach einem der Ansprüche 1 bis 4 als Texturbildner von kosmetischen oder pharmazeutischen Formulierungen.

6. Verwendung des homogenen Pulvers nach einem der Ansprüche 1 bis 4 in festen Formulierungen, insbesondere in Make-up-Grundlagen, Schminkpulvern, Wimperntusche oder Lippenstiften.

7. Verwendung des homogenen Pulvers nach einem der Ansprüche 1 bis 6 in flüssigen Formulierungen, insbesondere in Emulsionen, Lotionen oder Gelen, ganz besonders in zerstäubbaren Formulierungen oder auch in Lösungen, mit denen Gewebe oder Papier getränkt ist, insbesondere an Reinigungstüchlein oder Teintkorrekturpapier.
